# FASCICULE DE BREVET EUROPEEN

(11) **EP 0 890 651 B1**
(45) Date de publication et mention de la délivrance du brevet: **13.11.2002**
(21) Numéro de dépôt: 98401743.4
(22) Date de dépôt: 09.07.1998
(51) Int. Cl.: C12Q 1/68, G01N 33/487, G01N 21/66, G01N 33/53

(54) **Dispositif d'analyse à puce comprenant des électrodes a chauffage individuel**
IC-Vorrichtung zur Analyse, mit indivduell beheizbaren Elektroden
Analysing chip with individually heatable electrodes

(30) Priorité: 11.07.1997 FR 9708864
(43) Date de publication de la demande: 13.01.1999
(73) Titulaire: COMMISSARIAT A L'ENERGIE ATOMIQUE, 75015 Paris (FR)
(72) Inventeur: Caillat, Patrice, 38130 Echirolles (FR); Peltie, Philippe, 38760 Saint Paul de Varces (FR); Livache, Thierry, 38560 Haute Jarrie (FR)
(74) Mandataire: Dubois-Chabert, Guy

(56) Documents cités:
- EP-A- 0 640 828
- EP-A- 0 724 153
- WO-A-96/07917
- WO-A-96/28538

## Description

### Domaine technique

La présente invention concerne un dispositif d'analyse comprenant une puce électronique équipée d'une pluralité d'électrodes. Une telle puce est utilisée pour constituer une cellule d'analyse chimique ou biologique miniaturisée. En effet, chaque électrode de la puce peut être garnie individuellement d'un composé ou d'un matériau sensible à un produit chimique ou biologique déterminé.

Un dispositif d'analyse conforme à l'invention peut être utilisé, par exemple, comme mesureur de glucose, comme un dispositif d'analyse sanguine ou d'analyse de produits chimiques ou biologiques divers.

Une application particulière du dispositif de l'invention dans le domaine de la biologie moléculaire est celle de la reconnaissance anticorps-antigène. De la même façon, le dispositif peut aussi être utilisé dans les puces à ADN.

### Etat de la technique antérieure

Une puce électronique d'un dispositif d'analyse tel qu'évoqué ci-dessus peut comporter plusieurs centaines d'électrodes porteuses chacune d'une sonde ADN. On entend par sonde ADN des molécules d'ADN présentant une séquence prédéterminée et connue. L'hybridation sélective des molécules d'ADN se trouvant dans un milieu à analyser avec les molécules sondes fixées sur les électrodes permet de connaître la composition de ce milieu. A titre d'exemple, il est possible d'effectuer une analyse génétique sur des mutations responsables d'un type donné de cancer.

Bien entendu, dans d'autres applications d'analyse, les molécules sondes sont remplacées par tout réactif approprié sensible à une substance donnée, susceptible de se trouver dans le milieu à analyser.

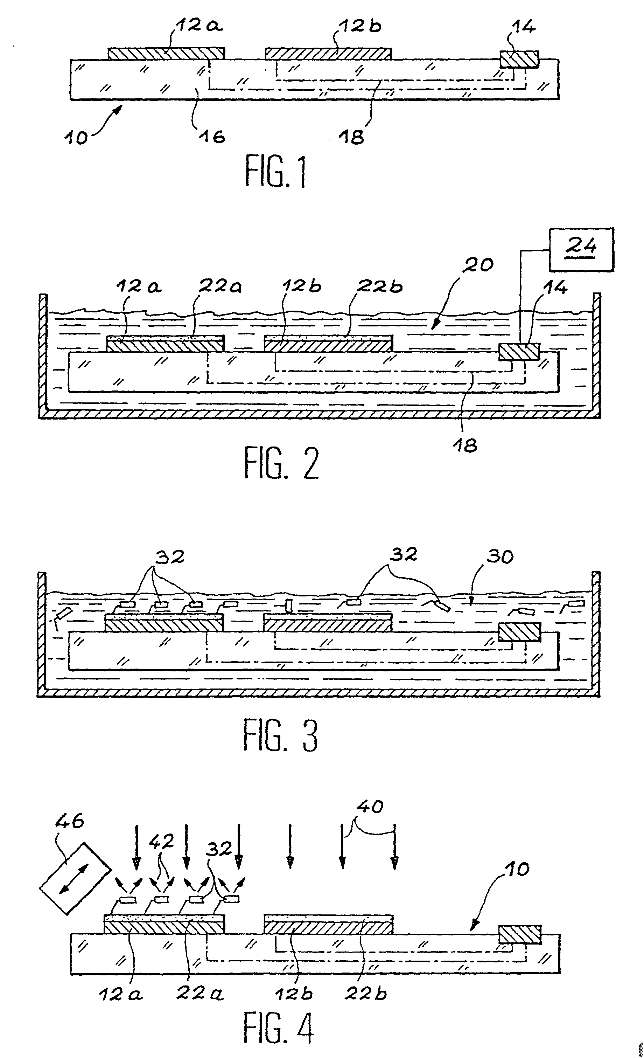

Les figures 1 à 4 annexées illustrent la structure et l'utilisation de puces à électrodes pour l'analyse d'un milieu.

La figure 1 montre en coupe et de façon schématique une puce 10 de capteur biologique tel qu'utilisé pour une reconnaissance anticorps-antigène ou comme sonde ADN-ADN d'un patient.

La puce 10 comporte des électrodes dites d'analyse 12a et 12b et des électrodes d'adressage 14. Bien que le nombre d'électrodes présentes sur une puce d'analyse est généralement élevé, de l'ordre d'une dizaine à plusieurs centaines, la puce 10 de la figure 1 n'est représentée qu'avec deux électrodes d'analyse et une seule électrode d'adressage, pour des raisons évidentes de clarté. Les électrodes d'analyse peuvent être adressées électriquement par des électrodes d'adressage correspondantes. Toutefois, lorsque le nombre d'électrodes d'analyse est très important, la puce peut comporter un dispositif d'adressage multiplexé permettant d'adresser l'ensemble des électrodes d'analyse à partir d'un nombre réduit d'électrodes d'adressage.

On entend par adressage la mise en liaison électrique d'une ou plusieurs électrodes d'analyse avec une ou plusieurs électrodes dites d'adressage ménagées généralement à la périphérie de la puce. Les électrodes d'adressage permettent d'appliquer ou de mesurer une tension sur les électrodes d'analyse, au moyen d'un appareil extérieur approprié.

Généralement, les électrodes d'analyse 12a et 12b sont réalisées en un métal tel que, par exemple, l'or, ou le platine. Elles sont mutuellement isolées les unes des autres sur une plaque de substrat 16. Des liaisons électriques entre les électrodes d'analyse 12a, 12b et les électrodes d'adressage sont ménagées dans le substrat 16 et sont très schématiquement indiquées avec la référence 18.

Une puce, telle que représentée à la figure 1, doit être configurée pour une utilisation particulière et les électrodes d'analyse sont à cet effet rendues fonctionnelles en les garnissant de molécules-sondes ou en recouvrant leur surface par un dépôt de réactif approprié.

Le dépôt de produits réactifs ou le greffage de molécules-sondes sur les électrodes est réalisé, en général, par électrodéposition.

Les réactifs ou molécules-sondes déposés sur les électrodes permettent, comme indiqué précédemment, un appariement sélectif avec des molécules spécifiques d'une substance à analyser. Ces molécules sont désignées par "molécules-cibles" dans la suite du texte.

La figure 2 montre la puce 10 plongée dans un bain d'électrolyte 20. On entend par bain d'électrolyte un bain approprié pour déposer, par électrochimie, un réactif sur les électrodes, ou un bain dans lequel sont diluées des molécules-sondes devant être fixées par électrodéposition sur les électrodes.

L'application sélective d'une tension de polarisation entre des électrodes d'analyse sélectionnées et une électrode de référence permet d'y fixer le produit réactif ou les molécules-sondes. La tension est appliquée aux électrodes au moyen d'un générateur extérieur 24 connecté aux électrodes d'adressage 14.

Les molécules-sondes sont fixées sur les électrodes d'analyse, par exemple, au moyen de polymères conducteurs de type polypyrrol ou polyaniline qui sont des porteurs de molécules-sondes.

La puce peut subir plusieurs étapes de dépôt électrochimique en étant trempée dans différents bains. Ainsi, différentes électrodes de la puce peuvent être recouvertes de différents réactifs, ou molécules-sondes, sensibles à différents composés des substances à analyser.

Sur la figure 2, on considère que les deux électrodes 12a et 12b sont respectivement (et successivement) recouvertes de réactifs, ou de molécules-sondes, différents 22a, 22b.

A l'issue du garnissage des électrodes avec les réactifs, de molécules-sondes, la puce est prête pour être utilisée afin d'analyser une substance, désignée par analyte dans la suite de la description.

Comme le montre la figure 3, la puce 10 est déconnectée du générateur et est plongée dans un bain 30 contenant l'analyte.

Ce bain contient, par exemple, des molécules-cibles 32 qui s'apparient ou réagissent avec le réactif ou les molécules-sondes 22a préalablement déposés sur la première électrode 12a. Pour des raisons de clarté, les molécules-cibles 32 sont représentées schématiquement et de façon grossie.

Les molécules-cibles 32 n'interagissent cependant pas avec la deuxième électrode 12b dont le revêtement réactif, ou les molécules-sondes, ne sont pas compatibles.

Après avoir été extraite du bain d'analyte 30, la puce est analysée afin de déterminer les électrodes pour lesquelles une réaction ou un appariement ont eu lieu.

Dans l'exemple décrit à la figure 4, on analyse la puce 10 par une méthode de détection par fluorescence. Une telle méthode est particulièrement adaptée lorsque les molécules-cibles 32 sont marquées par un produit de marquage fluorescent dit fluorophore.

Toutefois, d'autres méthodes d'analyse telles que des méthodes de mesure électrique par impédancemétrie, de mesure par microbalance, des mesures optiques par changement d'indice de réfraction, et des méthodes d'analyse par marquage radioactif sont également envisageables.

Comme le montre la figure 4, l'ensemble de la puce 10 est soumise à un rayonnement lumineux 40 d'une première longueur d'onde et provenant d'une source lumineuse non représentée.

Les molécules-cibles marquées absorbent le rayonnement lumineux 40 et émettent un rayonnement lumineux 42 avec une deuxième longueur d'onde caractéristique, différente de la première longueur d'onde.

Un dispositif de détection 46, sensible à la deuxième longueur d'onde permet de détecter la lumière réémise depuis les électrodes portant les molécules-cibles marquées par le produit fluorescent. Il est ainsi possible, en connaissant la nature des réactifs ou des molécules-sondes préalablement déposés sur chaque électrode de déterminer les composants de l'analyte qui s'y sont fixés.

Il ressort de la description qui précède qu'une étape cruciale pour l'analyse est l'étape dite d'appariement ou d'hybridation lors de laquelle les molécules cibles sont fixées sur les molécules sondes ou sur le réactif.

Pour garantir la fiabilité et la reproductibilité de l'appariement ou de l'hybridation pour des électrodes comportant les mêmes molécules sondes, il convient dans -un certain nombre de cas de chauffer et de maintenir à une température constante contrôlée la puce et l'analyte pendant l'appariement ou l'hybridation.

Toutefois, le chauffage à une température appropriée n'est possible que pour des puces sur lesquelles sensiblement toutes les électrodes sont garnies des mêmes molécules sondes. En effet, lorsque différents réactifs ou différentes molécules sondes disposées sur des électrodes d'une même puce nécessitent des températures d'hybridation ou d'appariement différentes, une analyse fiable n'est plus possible.

Un problème semblable se pose pour les puces d'analyse utilisées comme sonde ADN/ADN. Sur une telle puce, une molécule sonde d'ADN, bien identifiée est fixée sur chaque électrode.

Une pluralité de molécules sondes distinctes peuvent ainsi garnir les électrodes de la puce. Chaque molécule sonde comporte une successions de bases, repérées généralement avec les lettres A, T, C et G, dans un ordre déterminé.

Lorsque la puce est mise en contact avec l'analyte, un brin d'ADN contenu dans l'analyte peut s'apparier avec une molécule sonde donnée lorsqu'il présente une séquence de bases complémentaire à une séquence de base de la molécule sonde. Les liaisons possibles entre les bases sont de deux types : les liaisons hydrogène A-T (liaisons impliquant deux atomes d'hydrogène) et les liaisons hydrogène C-G (liaisons impliquant trois atomes d'hydrogène).

La réaction d'appariement, c'est-à-dire les liaisons entre bases complémentaires, est réversible. En chauffant les brins d'ADN à une température comprise généralement entre 30°C et 60°C, il est possible de casser les liaisons hydrogène.

Aussi, comme il existe deux types de liaisons possibles, indiquées ci-dessus, qui ne sont pas exactement identiques, il est possible de casser sélectivement les liaisons d'un type et non les liaisons de l'autre type.

Cette propriété est particulièrement importante pour supprimer des appariements accidentels ne correspondant pas à une séquence complète de bases complémentaires.

A titre d'exemple, lorsqu'une molécule sonde d'ADN comporte une séquence de 8 bases données, tout brin d'ADN de l'analyse comprenant une séquence complète ou partielle complémentaire des 8 bases peut s'apparier au moins en partie avec la molécule sonde. En chauffant à une température appropriée la structure appariée du brin d'ADN, il est possible d'éliminer sélectivement les appariements incomplets accidentels et de ne préserver que les appariements correspondant à la séquence complète complémentaire de la molécule sonde d'ADN.

Dans ce cas encore, il n'est pas possible de chauffer la puce à une température qui convient à l'ensemble des électrodes si celles-ci sont garnies de molécules sondes d'ADN présentant des séquences différentes. En effet, la température requise pour casser les liaisons d'une structure appariée dépend essentiellement de la première base de la séquence de la molécule sonde.

### Exposé de l'invention

La présente invention a pour but de proposer un dispositif d'analyse permettant d'effectuer un chauffage à une température désirée pour chaque électrode d'une puce, afin d'effectuer des analyses fiables et reproductibles avec des puces comprenant un grand nombre d'électrodes garnies de réactifs ou de molécules sonde différents.

Un but de l'invention est également de proposer un dispositif d'analyse à puce électronique permettant de contrôler précisément la température au voisinage de chaque électrode de la puce.

Un but de l'invention est aussi de proposer un dispositif d'analyse à puce économique et utilisable en particulier pour une analyse génétique.

Pour atteindre ces buts, l'invention a plus précisément pour objet un dispositif d'analyse qui comporte au moins une puce équipée d'une pluralité d'électrodes d'analyse, et qui comporte en outre des moyens de chauffage individuel des électrodes d'analyse.

Grâce aux moyens de chauffage individuel des électrodes d'analyse, il est possible, par exemple, de chauffer sélectivement un ensemble sélectionné d'électrodes d'analyse parmi la pluralité d'électrodes d'analyse.

Il est également possible, grâce au chauffage individuel, de porter chaque électrode à une température qui lui est propre.

Ainsi, dans le cas d'une puce comprenant des électrodes garnies de molécules sondes d'ADN, il est possible de chauffer chaque électrode à une température dépendant, par exemple, de la séquence d'ADN des molécules sondes dont elle est garnie.

Selon une première réalisation du dispositif de l'invention, les moyens de chauffage peuvent comporter, pour chaque électrode, une piste conductrice de chauffage disposée au voisinage de ladite électrode.

On considère que la piste conductrice de chauffage est disposée au voisinage d'une électrode d'analyse lorsque la distance moyenne séparant la piste conductrice de cette électrode est inférieure à la distance séparant la piste conductrice des autres électrodes d'analyse de la puce.

En particulier, chaque piste conductrice de chauffage peut être disposée de façon à entourer l'électrode.

Selon une variante, dans laquelle les électrodes sont elles-mêmes configurées sous la forme de pistes conductrices, une piste conductrice de chauffage peut être respectivement associée à au moins une piste conductrice formant électrode et s'étendant alors parallèlement à celle-ci.

Les moyens de chauffage individuel peuvent comporter des circuits électroniques d'adressage reliés à des électrodes d'adressage, et conçus pour faire passer sélectivement dans chaque piste conductrice de chauffage un courant correspondant au chauffage local souhaité.

L'alimentation en courant des pistes conductrices de chauffage peut être directe, en utilisant un nombre de circuits et d'électrodes d'adressage correspondant au nombre de pistes conductrices de chauffage. L'alimentation en courant peut aussi avoir lieu par l'intermédiaire de circuits multiplexés.

Selon une autre réalisation particulière du dispositif de l'invention, dans laquelle la puce comporte des pistes conductrices formant des électrodes d'analyse, les moyens de chauffage peuvent comporter au moins un circuit électronique d'adressage pour appliquer sélectivement et individuellement un courant de chauffage aux pistes conductrices formant des électrodes d'analyse. Ainsi, les pistes ont une double fonction d'électrode d'analyse et de chauffage.

Une telle mesure permet de réduire l'encombrement et le coût des moyens de chauffage.

Selon un aspect avantageux de l'invention, le dispositif d'analyse de l'invention peut également comporter des moyens d'isolation thermique mutuel des électrodes et des pistes conductrices de chauffage.

On entend par isolation thermique mutuel des électrodes une isolation permettant de réduire les échanges thermiques entre électrodes voisines et entre une électrode donnée et une piste conductrice de chauffage associée à une électrode voisine de ladite électrode donnée.

Les moyens d'isolation thermique peuvent en particulier comporter une couche de matériau polymère, isolante électrique, disposée sur la puce, et sur laquelle sont formées les électrodes d'analyse. Cette couche de matériau polymère est de préférence choisie avec une conductibilité thermique inférieure à celle de la puce.

Selon une variante de mise en oeuvre de l'invention, les moyens de chauffage individuel des électrodes d'analyse peuvent aussi comporter au moins une source laser et au moins un réseau de microlentilles pour faire converger un rayonnement de chauffage vers des électrodes d'analyse sélectionnées de ladite pluralité d'électrode d'analyse.

Afin d'effectuer un chauffage sélectif et dosé pour chacune des électrodes d'analyse, le réseau de microlentilles peut être pourvu d'un masque présentant des régions de densité variable et disposés devant les microlentilles.

En particulier, le réseau de microlentilles peut être équipé d'un masque présentant des régions transparentes et des régions opaques. Le masque peut également comporter des régions laissant plus ou moins passer la lumière du laser.

D'autres caractéristiques et avantages de l'invention ressortiront mieux de la description qui va suivre, en référence aux figures des dessins annexés. Cette description est donnée à titre purement illustratif et non limitatif.

### Brève description des figures

- La figure 1, déjà décrite, est une coupe schématique d'une puce de capteur biologique.
- La figure 2, déjà décrite, est une coupe schématique de la puce de la figure 1 plongée dans un bain d'électrolyte.
- La figure 3, déjà décrite, est une coupe schématique de la puce de la figure 1, plongée dans un bain d'analyte.
- La figure 4, déjà décrite, est une coupe schématique de la puce de la figure 1, soumise à une analyse par fluorescence.
- La figure 5, est une vue schématique de dessus d'une puce électronique telle qu'utilisée dans un dispositif d'analyse conforme à l'invention.
- La figure 6 est une vue à échelle agrandie illustrant la réalisation particulière d'une électrode d'analyse de la puce électronique et d'une piste de chauffage associée.
- La figure 7 est une vue à échelle agrandie illustrant une autre réalisation possible d'une électrode d'analyse de la puce électronique et de la piste de chauffage associée.
- La figure 8 est une vue à échelle agrandie illustrant encore une autre réalisation particulière d'une électrode d'analyse de la puce électronique.
- La figure 9 est une coupe schématique locale de la puce à échelle agrandie, passant par une électrode d'analyse conforme à la figure 8.
- La figure 10 est une coupe schématique locale de la puce à échelle agrandie, passant par une électrode d'analyse et une piste conductrice de chauffage, et illustrant la structure de la puce selon une réalisation particulière.
- La figure 11 est une coupe schématique locale de la puce à échelle agrandie, passant par une électrode d'analyse et une piste conductrice de chauffage et illustrant une structure de la puce constituant une variante par rapport à la structure de la figure 10.
- La figure 12 est une vue schématique d'une variante du dispositif d'analyse conforme à l'invention.
- Les figures 13 et 14 sont des vues schématiques illustrant deux réalisations particulières du dispositif de la figure 12.

### Description détaillée de modes de mise en oeuvre de l'invention

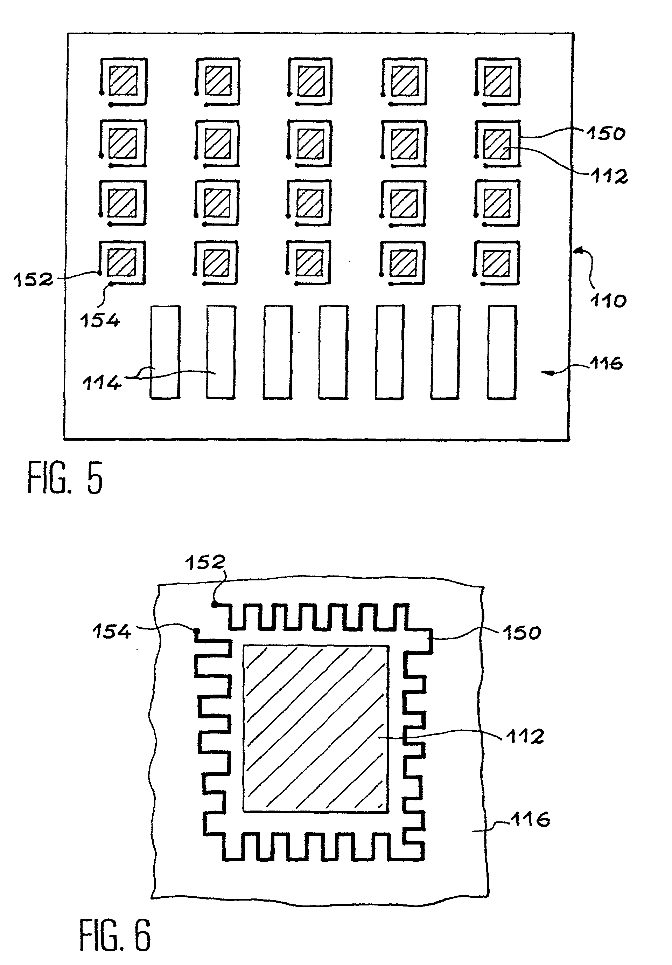

La figure 5 montre une puce électronique conforme à l'invention utilisée dans un dispositif d'analyse.

La puce, réalisée sur un substrat 116 présente une pluralité d'électrodes d'analyse 112 réalisées sous la forme de pastilles métalliques, par exemple des pastilles d'or ou de platine. Chaque électrode peut comporter une ou plusieurs pastilles métalliques mutuellement isolées.

Sur la figure 5, chaque électrode comporte une seule pastille métallique.

La puce comporte également, dans une région périphérique du substrat, des électrodes d'adressage 114 susceptibles d'être raccordées à des appareillages électriques extérieurs par l'intermédiaire d'un connecteur approprié non représenté. Les appareillages électriques extérieurs, également non représentés, peuvent comporter des sources de courant et/ou de tension pour permettre un dépôt électrochimique de matériaux réactifs ou de molécules sondes sur les électrodes d'analyse. Les appareillages électriques peuvent également comporter des moyens de mesure, par exemple, pour mesurer des impédances entre des électrodes.

Pour mettre en liaison électrique les électrodes d'analyse et les électrodes d'adressage, la puce peut comporter, par exemple, un ensemble de pistes conductrices intégrées formant des circuits de liaison. La puce peut également comporter des circuits électroniques à multiplexeur pour adresser un grand nombre d'électrodes d'analyse à partir d'un nombre réduit d'électrodes d'adressage.

On peut noter que sur la figure 5 que chaque électrode d'analyse 112 est entourée par une spire sous la forme d'une piste conductrice 150. La piste conductrice 150 constitue une résistance chauffante qui, lorsqu'elle est parcourue par un courant, permet d'élever la température de l'électrode. Elle permet également de chauffer localement un analyte ou un électrolyte qui serait en contact avec l'électrode.

De façon avantageuse, un certain nombre d'électrodes d'adressage 114 peut également être prévu pour sélectivement appliquer à chaque piste conductrice 150 un courant approprié au chauffage local souhaité. Dans ce cas également, l'application du courant à chaque piste conductrice peut avoir lieu par une pluralité de circuits électriques indépendants ou par un circuit du type multiplex.

Ces circuits sont reliés par des connexions internes non représentées sur la figure, aux bornes 152 et 154 de chaque piste conductrice 150.

Le courant nécessaire pour le chauffage peut être appliqué par une source de courant appropriée extérieure à la puce et reliée aux électrodes d'adressages par un connecteur correspondant.

Selon un aspect particulier avantageux, chaque piste conductrice de chauffage peut également être utilisé comme contre-électrode. Elle est alors utilisée lors d'un traitement électrochimique pour le garnissage des électrodes avec un réactif ou des molécules sondes. Dans ce cas, la piste conductrice est également adressable de façon sélective pour lui appliquer un potentiel électrique approprié à l'électrodéposiotion.

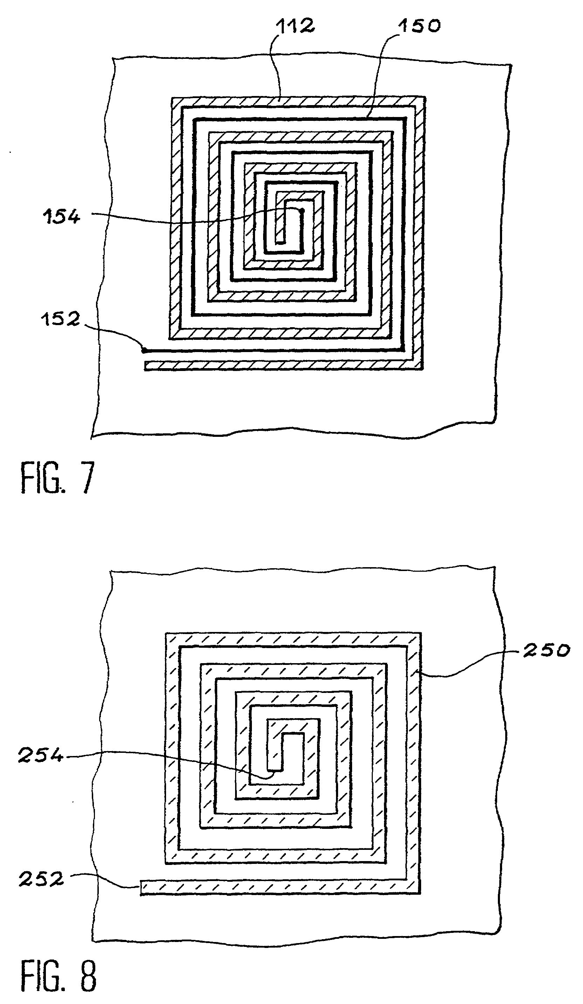

La figure 6 montre, a plus grande échelle, une configuration particulière d'une électrode d'analyse et d'une piste conductrice de chauffage qui lui est associée.

L'électrode d'analyse 112, sous la forme d'une pastille métallique carrée, est entourée par une piste conductrice qui présente sur son trajet, le long des côtés de l'électrode, un motif en créneaux. Ce motif permet d'augmenter la longueur de la piste conductrice 150 et aussi de favoriser le chauffage de l'électrode.

Comme pour la figure 5, les références 152 et 154 désignent les bornes de la piste conductrice 150.

Une autre possibilité encore de configuration des électrodes et des pistes conductrices de chauffage est représentée à la figure 7.

Selon cette configuration, l'électrode d'analyse 112 se présente également sous la forme d'une piste conductrice.

La piste conductrice formant l'électrode d'analyse et la piste conductrice de chauffage s'étendent sensiblement parallèlement et sont repliées, dans cet exemple, en forme de spirale carrée.

Les bornes de la piste conductrice de chauffage sont indiquées avec les références 152 et 154.

La figure 8 montre encore un autre mode de réalisation de l'électrode et de la piste de chauffage.

Conformément à ce mode de réalisation, une unique piste conductrice 250 forme à la fois l'électrode d'analyse et le moyen de chauffage de cette électrode. La piste conductrice 250 est repliée en forme de spirale carrée. Des bornes terminales de la piste dont indiquées avec les références 252 et 254.

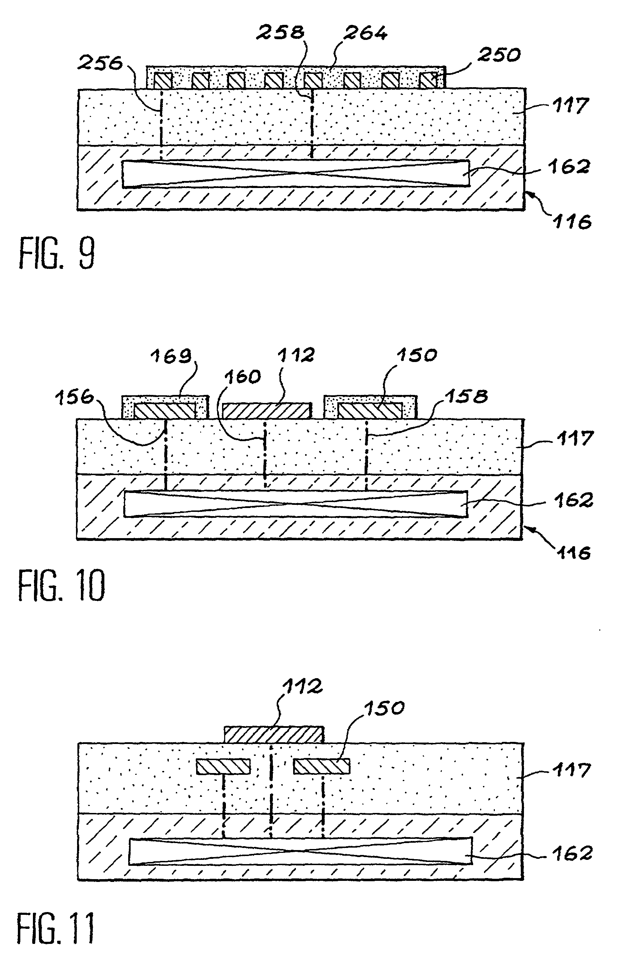

La figure 9 est une coupe schématique locale et partielle d'un substrat comprenant des électrodes conformes à celle représentée à la figure 8.

Sur la figure 9, on observe que la puce présente une surface recouverte par une couche 117 en un matériau isolant électrique. Il s'agit par exemple d'une couche de polyimide d'une épaisseur de l'ordre de 15 µm.

La piste conductrice 250 est formée à la surface de la couche isolante 117. Deux lignes de connexion indiquées très schématiquement avec les références 256 et 258 relient respectivement les bornes 252 et 254 de la piste conductrice à un dispositif d'adressage 162 de la puce, représenté également de façon très schématique. Ce dispositif d'adressage permet, comme décrit ci-dessus, d'appliquer sélectivement un courant de chauffage à chaque piste conductrice, par l'intermédiaire des électrodes d'adressage.

Le matériau utilisé pour former la couche 117 est préférentiellement un matériau polymère présentant des propriétés d'isolation thermique. Ainsi, la piste conductrice est isolée thermiquement, non seulement du substrat 116 de la puce, mais également des pistes conductrices formant les électrodes conductrices voisines. Cette isolation thermique permet de rendre encore plus indépendant le chauffage de chaque électrode individuelle.

Par ailleurs, la piste conductrice 250 peut être recouverte d'une fine couche 264 en un matériau isolant électrique. Ainsi, un substrat équipé de telles électrodes peut être mis en oeuvre pour l'analyse d'analytes électriquement conducteurs.

Dans ce cas cependant, comme la piste 250 est isolée, le garnissage avec un réactif n'est pas effectué par voie électrochimique mais par exemple avec des micro-pipettes.

Les figures 10 et 11 montrent également des coupes de puces selon d'autres modes de réalisation dans lesquelles l'électrode d'analyse est distincte de la piste conductrice de chauffage.

Sur la puce de la figure 10, la piste conductrice de chauffage 150 est disposée autour de l'électrode d'analyse 112. Elle est, tout comme l'électrode 112 elle-même, formée sur une couche de matériau isolant électrique 117 qui recouvre le substrat 116 de la puce.

Comme indiqué précédemment, le matériau isolant électrique peut être choisi également en fonction de ses propriétés d'isolation thermique. Ainsi, la piste conductrice de chauffage, placée au voisinage immédiat d'une électrode correspondante permet l'élévation en température de cette électrode sans influer de façon significative sur des électrodes voisines.

Sur la figure 10, les références 156, 158 et 160 indiquent de façon très schématique des liaisons de connexion électrique reliant respectivement les bornes de la piste conductrice de chauffage et l'électrode d'analyse à des circuits d'adressage 162, représentés également de façon schématique.

Une fine couche de matériau isolant électrique 164 peut être formée sur la piste conductrice de chauffage 150. Cette couche de matériau isolant électrique permet d'éviter tout court-circuit ou interaction avec un analyte mis en contact avec la puce. Le rôle isolant de cette couche est particulièrement avantageux lorsque l'électrolyte utilisé est très conducteur.

La figure 11 montre une variante selon laquelle la piste conductrice de chauffage 150, associée à une électrode d'analyse 112, est enterrée dans la couche de matériau isolant électrique 117. La piste conductrice de chauffage 150 est disposée, au moins en partie, sous l'électrode 112 correspondante. Elle occupe ainsi moins de place à la surface de la puce.

Comme la piste conductrice de chauffage est enterrée dans le matériau isolant électrique 117, elle est automatiquement isolée électriquement à la fois de l'électrode d'analyse 112 et des analytes avec lesquels la puce est susceptible d'être mise en contact..

La piste conductrice de chauffage est enterrée de préférence à une faible distance sous la surface de la couche d'isolant électrique, de façon à permettre une conduction thermique suffisante vers l'électrode d'analyse.

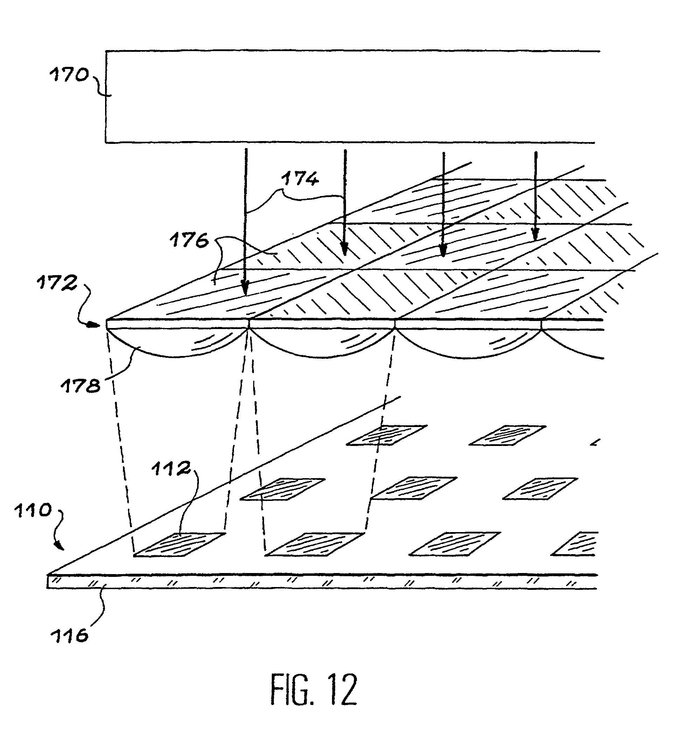

La figure 12 montre une deuxième mise en oeuvre de l'invention qui ne nécessite pas de pistes conductrices de chauffage ni d'adressage électrique.

La puce 110 utilisée pour cette mise en oeuvre de l'invention comporte simplement un substrat isolant 116 sur lequel sont formées des électrodes d'analyse 112. Accessoirement, le substrat peut également comporter des contre-électrodes, et des électrodes d'adressage telles que décrites précédemment. Il peut aussi être garni d'une couche d'isolation thermique permettant de réduire les échanges thermiques entre les électrodes.

Les moyens de chauffage individuel des électrodes d'analyse 112 comportent une source laser 170 et un réseau 172 de microlentilles.

Le réseau 172 de microlentilles reçoit un faisceau lumineux 174 en provenance du laser. Il est disposé en face des électrodes d'analyse 112 de la puce 110 de façon à concentrer le faisceau lumineux 174 sélectivement sur les électrodes 112, comme indiqué sur la figure 12.

Le faisceau lumineux concentré sur les électrodes permet de les chauffer.

A titre d'exemple pour une électrode d'analyse carrée de 50 µm de côté, exposée à un faisceau laser d'une longueur d'onde de 0,5 µm pendant 1 ns, il est nécessaire, pour obtenir à sa surface une élévation de température de 50°C, de lui appliquer une puissance instantanée de l'ordre de 395 mW.

En un temps aussi bref, la profondeur de pénétration de la chaleur dans l'électrode est de l'ordre de 10 nm. La chaleur n'a donc pas le temps d'être évacuée par l'électrode.

La source laser 170 utilisée est, par exemple, un microlaser pulsé émettant dans le vert et présentant une puissance de crête de l'ordre de 200 W à 800 W.

Le réseau de microlentilles peut comporter un nombre de microlentilles correspondant aux électrodes à illuminer et donc à chauffer.

Le réseau de microlentilles peut également comporter un masque avec des régions 176 de densité variable agencées de façon à coïncider avec des microlentilles 178 du réseau.

Ainsi, le choix du masque et l'agencement des régions 176, plus ou moins opaques, permet le chauffage sélectif approprié des électrodes d'analyse correspondantes.

En effet, on peut montrer que pour des microlentilles présentant une dimension de 2d et agencée, selon un pas de 2a, la distribution d'intensité I(x) due à la diffraction de la lumière cohérente du laser est donnée par la formule suivante : I(x)=[f(dx)/dx]²[sin(Nax)/Nax]², ou x représente le pas angulaire.

Le premier terme de l'expression ci-dessus représente l'enveloppe. La fonction f(u) est un sinus si la pupille de la microlentille est carrée, et une fonction de Bessel si la pupille est circulaire.

Le second terme représente la contribution de N microlentilles constituant le réseau. Il convient de noter que les intensités des différents points lumineux obtenus n'est pas uniforme. En effet, les microlentilles sont de grand diamètre par rapport au pas des électrodes d'analyse et à la longueur d'onde du laser. A cet effet s'ajoute la répartition énergétique de lumière gaussienne due au laser.

Un masque de densité variable placé devant les microlentilles 178 permet si nécessaire de corriger la non uniformité de l'intensité. Il est également possible de ne pas utiliser de masque et de mettre à profit la non-uniformité pour éclairer et donc chauffer différemment diverses électrodes d'analyse de la puce.

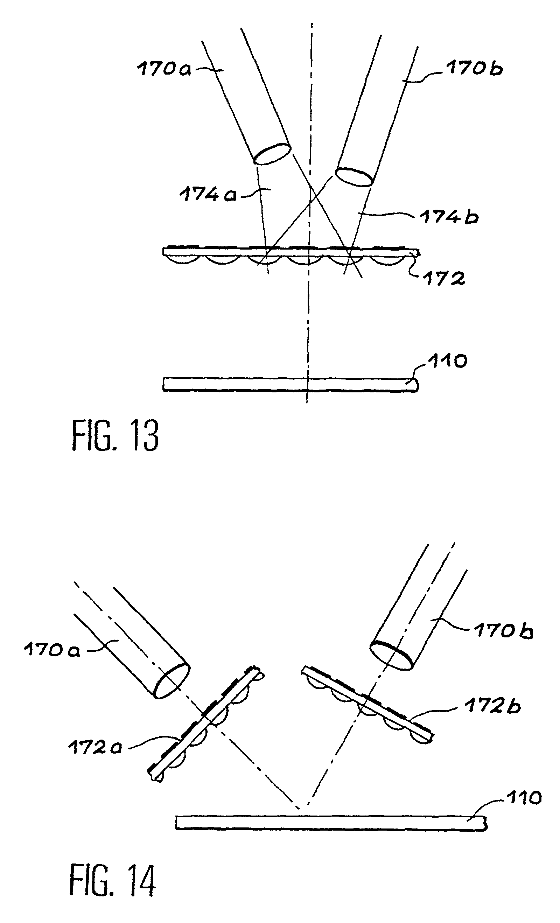

Comme le montrent les figures 13 et 14, plusieurs sources laser peuvent être utilisées.

Sur la figure 13, la puce 110 est éclairée et chauffée par deux lasers 170a, 170b.

Les faisceaux 174a, 174b des lasers 170a et 170b traversent un réseau 172 de microlentilles disposé de façon sensiblement parallèle à la surface de la puce 110.

On peut noter que les faisceaux 174a et 174b ne sont pas perpendiculaires au réseau 172 de microlentilles et à la puce 110. Les lasers 170a et 170b sont disposés symétriquement par rapport à un axe perpendiculaire au plan du réseau 172 de microlentilles et au plan de la surface de la puce 110, et forment un angle par rapport à cet axe.

Selon une variante, représentée schématiquement à la figure 14, deux réseaux distincts 172a et 172b de microlentilles sont utilisés, chaque réseau étant disposé devant un laser 170a, 170b.

Chaque réseau est disposé sensiblement perpendiculairement à l'axe du faisceau de lumière émis par le laser correspondant. Par ailleurs, les lasers 170a et 170b sont orientés symétriquement de façon que leurs faisceaux forment un angle par rapport à un axe perpendiculaire à la surface de la puce 110.

Une combinaison adaptée d'un ou plusieurs lasers et de un ou plusieurs réseaux de microlentilles permet comme indiqué précédemment de chauffer différemment diverses électrodes d'analyse de la puce.

Grâce à cette caractéristique la puce peut être une puce plus ordinaire ne comportant pas de pistes conductrices de chauffage associées aux électrodes.

La mise en oeuvre de l'invention conforme aux figures 12 à 14, utilisant un chauffage par laser, peut avantageusement être combinée à une méthode d'analyse mettant en oeuvre un marquage fluorescent de molécules cibles. Une telle méthode est décrite dans la partie introductive du texte. En effet, le faisceau de chauffage peut être mis à profit pour exciter les fluorophores des molécules marquées.

## Revendications

1. Dispositif d'analyse comportant au moins une puce (110) équipée d'une pluralité d'électrodes d'analyse (112) et **caractérisé en ce qu'**il comporte en outre des moyens de chauffage individuel (150, 170) des électrodes d'analyse.

2. Dispositif d'analyse selon la revendication 1, dans lequel les moyens de chauffage comportent, pour chaque électrode d'analyse une piste conductrice (150, 250) de chauffage disposée au voisinage de ladite électrode.

3. Dispositif d'analyse selon la revendication 2, dans lequel la piste conductrice de chauffage (150) présente un motif en serpentin.

4. Dispositif d'analyse selon la revendication 2, dans lequel la piste conductrice (150) entoure ladite électrode (112).

5. Dispositif selon la revendication 2, dans lequel la piste conductrice de chauffage (150) de chaque électrode d'analyse forme une contre-électrode associée à ladite électrode d'analyse.

6. Dispositif d'analyse selon la revendication 2 dans lequel la piste conductrice (150) est entourée d'un matériau isolant électrique (169).

7. Dispositif selon la revendication 1 comprenant des moyens d'isolation thermique (117) mutuel des électrodes d'analyse.

8. Dispositif selon la revendication 7, dans lequel les moyens d'isolation thermique des électrodes comportent une couche (117) de polymère disposée sur la puce, les électrodes d'analyse (112) et les pistes conductrices de chauffage (150) étant formées sur ladite couche de polymère.

9. Dispositif selon la revendication 7, dans lequel les moyens d'isolation thermique des électrodes comportent une couche (117) de polymère disposée sur la puce, les électrodes d'analyse (112) étant formées sur ladite couche de polymère et les pistes conductrices de chauffage (150) étant enterrées dans la couche de polymère, respectivement au voisinage des électrodes correspondantes.

10. Dispositif selon la revendication 2 comportant des pistes conductrices (112) formant des électrodes d'analyse, une piste conductrice de chauffage étant respectivement associée à au moins une piste conductrice formant électrode d'analyse et s'étendant parallèlement à celle-ci.

11. Dispositif selon la revendication 1, dans lequel la puce comporte des pistes conductrices (250) formant des électrodes d'analyse et dans lesquels les moyens de chauffage comportent au moins un circuit électronique d'adressage (162) pour appliquer sélectivement et individuellement un courant de chauffage aux pistes conductrices formant des électrodes d'analyse.

12. Dispositif selon la revendication 11, les pistes conductrices formant des électrodes d'analyse (250) présentent un motif en serpentin et sont recouvertes par une couche de matériau isolant électrique (264).

13. Dispositif selon la revendication 1, dans lequel les moyens de chauffage individuel de chaque électrode d'analyse comportent au moins une source laser (170, 170a, 170b) et au moins un réseau de microlentilles (172, 172a, 172b) pour faire converger un rayonnement de chauffage vers des électrodes d'analyse (112) sélectionnées de ladite pluralité d'électrode d'analyse.

14. Dispositif selon la revendication 13, dans lequel le réseau de microlentille (172)est pourvu d'un masque (176) présentant des régions de densité variable, et disposées devant les microlentilles.

15. Dispositif d'analyse selon la revendication 1, dans laquelle la puce est équipée d'électrodes d'adressage (114) pour appliquer sélectivement sur chaque électrode d'analyse (112) une tension dite tension d'adressage.

16. Dispositif d'analyse selon la revendication 1, dans laquelle la puce est équipée d'électrodes d'adressage pour mesurer une impédance entre chaque électrode d'analyse et au moins une contre-électrode.

17. Dispositif selon la revendication 15 ou 16 dans lequel les électrodes d'analyse et les électrodes d'adressage sont reliées par un circuit électronique multiplexe.

18. Puce électronique comprenant une pluralité d'électrodes (112) dites électrodes d'analyse, **caractérisée en ce que** la puce comporte en outre, pour chaque électrode d'analyse, une piste conductrice de chauffage (150) disposée au voisinage de ladite électrode d'analyse.

19. Puce électronique selon la revendication 18, comportant en outre des électrodes dites électrodes d'adressage (114) et des moyens. (162) à circuits électroniques reliant les électrodes d'adressage aux électrodes d'analyse et aux pistes conductrices de chauffage.

20. Puce électronique selon la revendication 18, comprenant en outre une contre-électrode.

## Patentansprüche

1. Analysevorrichtung mit wenigstens einem IC (110), ausgestattet mit einer Vielzahl Analyseelektroden (112) und
**dadurch gekennzeichnet, dass** sie außerdem individuelle Heizeinrichtungen (150, 170) der Analyseelektroden umfasst.

2. Analysevorrichtung nach Anspruch 1, bei der die Heizeinrichtungen jeder Analyseelektrode eine in der Nähe der genannten Elektrode befindliche Heizleiterbahn (150, 250) umfasst.

3. Analysevorrichtung nach Anspruch 2, bei der die Heizleiterbahn (150) ein serpentinenförmiges Muster aufweist.

4. Analysevorrichtung nach Anspruch 2, bei der die Heizleiterbahn (150) die genannte Elektrode (112) umgibt.

5. Analysevorrichtung nach Anspruch 2, bei der die Heizleiterbahn (150) jeder Analyseelektrode eine dieser Analyseelektrode zugeordnete Gegenelektrode bildet.

6. Analysevorrichtung nach Anspruch 2, bei der die Heizleiterbahn (150) von einem elektrisch isolierenden Material (169) umgeben ist.

7. Vorrichtung nach Anspruch 1 mit Einrichtungen (117) zur gegenseitigen Wärmeisolation der Analyseelektroden.

8. Vorrichtung nach Anspruch 7, bei der die Einrichtungen zur Wärmeisolation der Elektroden eine auf dem IC angeordnete Polymerschicht (117) umfassen, wobei die Analyseelektroden (112) und die Heizleiterbahnen (150) auf der genannten Polymerschicht ausgebildet sind.

9. Vorrichtung nach Anspruch 7, bei der die Einrichtungen zur Wärmeisolation der Elektroden eine auf dem IC angeordnete Polymerschicht (117) umfassen, wobei die Analyseelektroden (112) auf der genannten Polymerschicht ausgebildet sind und die Heizleiterbahnen (150) in der Polymerschicht vergraben sind, jeweils in der Nähe der entsprechenden Elektroden.

10. Vorrichtung nach Anspruch 2, Leiterbahnen (112) umfassend, die Analyseelektroden bilden, wobei eine Heizleiterbahn jeweils wenigstens einer eine Analyseelektrode bildenden Leiterbahn zugeordnet ist und sich parallel zu dieser erstreckt.

11. Vorrichtung nach Anspruch 1, bei der der IC Leiterbahnen (250) umfasst, die Analyseelektroden bilden, und bei der die Heizeinrichtungen wenigstens eine elektronische Adressierschaltung (162) umfassen, um einen Heizstrom selektiv und individuell in die Leiterbahnen einzuspeisen, die Analyseelektroden bilden.

12. Vorrichtung nach Anspruch 11, wobei die Leiterbahnen, die Analyseelektroden (250) bilden, ein serpentinenförmiges Muster aufweisen und mit einer Schicht aus elektrisch isolierendem Material (264) überzogen sind.

13. Vorrichtung nach Anspruch 1, bei der die individuellen Heizeinrichtungen jeder Analyseelektrode wenigstens eine Laserquelle (170, 170a, 170b) und wenigstens ein Mikrolinsengitter (172, 172a, 172b) umfassen, um eine Heizstrahlung auf Analyseelektroden zu konvergieren, die aus der Vielzahl von Analyseelektroden ausgewählt werden.

14. Vorrichtung nach Anspruch 13, bei der das Mikrolinsengitter (172) mit einer Maske (176) versehen ist, die Bereiche variabler Dichte aufweist und vor den Mikrolinsen angeordnet ist.

15. Vorrichtung nach Anspruch 1, bei der der IC mit Adressierelektroden (114) ausgestattet ist, um an jede Analyseelektrode selektiv eine Adressierspannung genannte Spannung anlegen zu können.

16. Vorrichtung nach Anspruch 1, bei der der IC mit Adressierelektroden ausgerüstet ist, um zwischen jeder Analyseelektrode und wenigstens einer Gegenelektrode eine Impedanz zu messen.

17. Vorrichtung nach Anspruch 15 oder 16, bei der die Analyseelektroden und die Adressierelektroden durch eine elektronische Multiplexschaltung verbunden sind.

18. Elektronischer Baustein bzw. IC mit einer Vielzahl Elektroden (112), Analyseelektroden genannt,
**dadurch gekennzeichnet,**
**dass** dieser IC außerdem für jede Analyseelektrode eine in der Nähe dieser Analyseelektrode befindliche Heizleiterbahn (150) umfasst.

19. Elektronischer Baustein bzw. IC nach Anspruch 18, der außerdem Adressierelektroden genannte Elektroden (114) und Einrichtungen (162) mit elektronischen Schaltungen umfasst, die die Adressierelektroden mit den Analyseelektroden und mit den Heizleiterbahnen verbinden.

20. Elektronischer Baustein bzw. IC nach Anspruch 18, der außerdem eine Gegenelektrode umfasst.

## Claims

1. An analysis device including at least one chip (110) equipped with a plurality of analysis electrodes (112), and **characterized in that** it also includes means of individual heating (150, 170) of the analysis electrodes.

2. An analysis device according to claim 1, in which the heating means include, for each analysis electrode, a conductive heating track (150, 250) disposed in the vicinity of the said electrode.

3. An analysis device according to claim 2, in which the conductive heating track (150) has the pattern of a coil.

4. An analysis device according to claim 2, in which the conductive track (150) surrounds said electrode (112).

5. A device according to claim 2, in which the conductive heating track (150) of each analysis electrode forms a counter electrode associated with said analysis electrode.

6. An analysis device according to claim 2, in which the conductive track (150) is surrounded by an electrically isolating material (169).

7. A device according to claim 1, comprising means of mutual thermal isolation (117) of the analysis electrodes.

8. A device according to claim 7, in which the means of thermal isolation of the electrodes include a layer (117) of polymer disposed on the chip, the analysis electrodes (112) and the conductive heating tracks (150) being formed on said polymer layer.

9. A device according to claim 7, in which the means of thermal isolation of the electrodes consist of a layer (117) of polymer disposed on the chip, the analysis electrodes (112) being formed on said polymer layer and the conductive heating tracks (150) being buried in the polymer layer, respectively in the vicinity of the corresponding electrodes.

10. A device according to claim 2, including conductive tracks (112) forming analysis electrodes, a conductive heating track being respectively associated with at least one conductive track forming an analysis electrode and extending parallel to it.

11. A device according to claim 1, in which the chip includes conductive tracks (250) forming analysis electrodes and in which the heating means include at least one electronic addressing circuit (162) for selectively and individually applying a heating current to the conductive tracks forming analysis electrodes.

12. A device according to claim 11, the conductive tracks forming analysis electrodes (250) have the pattern of a coil and are covered with a layer of electrically isolating material (264).

13. A device according to claim 1, in which the means of individual heating of each analysis electrode include at least one laser source (170, 170a, 170b) and at least one network of microlenses (172, 172a, 172b) in order to make heating radiation converge towards selected analysis electrodes (112) of said plurality of analysis electrodes.

14. A device according to claim 13, in which the network of microlenses (172) is provided with a mask (176) having regions of variable density and disposed in front of the microlenses.

15. An analysis device according to claim 1, in which the chip is equipped with addressing electrodes (114) for selectively applying to each analysis electrode (112) a voltage referred to as an addressing voltage.

16. An analysis device according to claim 1, in which the chip is equipped with addressing electrodes for measuring an impedance between each analysis electrode and at least one counter electrode.

17. A device according to claim 15, in which the analysis electrodes and the addressing electrodes are connected by a multiplex electronic circuit.

18. An electronic chip comprising a plurality of electrodes (112) referred to as analysis electrodes, **characterized in that** the chip also has, for each analysis electrode, a conductive heating track (150) disposed in the vicinity of said analysis electrode.

19. An electronic chip according to claim 18, also including electrodes referred to as addressing electrodes (114) and electronic circuit means (162) connecting the addressing electrodes to the analysis electrodes and to the conductive heating tracks.

20. An electronic chip according to claim 18, also comprising a counter electrode.
